# EUROPÄISCHE PATENTSCHRIFT

(11) **EP 3 841 380 B1**
(45) Veröffentlichungstag und Bekanntmachung des Hinweises auf die Patenterteilung: **05.10.2022**
(21) Anmeldenummer: 18762041.4
(22) Anmeldetag: 21.08.2018
(51) Int. Cl.: G01N 33/42, E01C 19/28

(54) **SYSTEM ZUR VERDICHTUNGSMESSUNG**
SYSTEM FOR MEASURING COMPACTION
SYSTÈME DE MESURE DE COMPACTAGE

(43) Veröffentlichungstag der Anmeldung: 30.06.2021
(73) Patentinhaber: MOBA Mobile Automation AG, 65555 Limburg (DE)
(72) Erfinder: MARX, Bernhard, 65555 Limburg (DE)
(74) Vertreter: Pfitzner, Hannes
(86) Internationale Anmeldenummer: PCT/EP2018/072563
(87) Internationale Veröffentlichungsnummer: WO 2020/038567

(56) Entgegenhaltungen:
- EP-A1- 3 147 406
- WO-A1-2018/019408
- DE-A1-102017 107 145
- DE-U1-202009 008 592
- Lemon Bob ET AL: "Final Intelligent Asphalt Compaction Analyzer", , 13 November 2016 (2016-11-13), pages 1-78, XP055895342, Retrieved from the Internet: URL:https://web.archive.org/web/20161113/h ttps://www.fhwa.dot.gov/hfl/partnerships/i aca/final_report.pdf [retrieved on 2022-02-24]

## Beschreibung

Ausführungsbeispiele der vorliegenden Erfindung beziehen sich auf ein System zur Verdichtungsmessung sowie auf eine Baumaschine, wie eine Verdichtungsmaschine mit einem entsprechenden System. Weitere Ausführungsbeispiele beziehen sich auf ein Verfahren zur Verdichtungsmessung sowie auf ein entsprechendes Computerprogramm.

Im Allgemeinen liegt die Erfindung auf dem Gebiet von Walzen, beispielsweise Straßenwalzen zum Verdichten einer Straßen- oder Fahrbahnoberfläche. Bevorzugte Ausführungsbeispiele beziehen sich auf ein Verdichtungsmesssystem für eine durch eine Walze zu verdichtende Materialschicht, wie beispielsweise Erd- oder Asphaltschicht.

Als Resultat der Verdichtungsarbeit einer Walze ist in der Regel ein homogenes und ausreichend verdichtetes Feld erwünscht, denn dieses ist die Voraussetzung für hochtragfähige Böden und langlebige Straßenbeläge.

Im Straßenbau ist es wichtig, dass der von Straßenfertigern aufgebrachte Straßenbelag, wie beispielsweise Asphaltmaterial, anschließend von Straßenwalzen verdichtet wird. Dazu bewegen sich eine oder mehrere Straßenwalzen während des Arbeitseinsatzes in einem von einem oder mehreren Straßenfertigern vorgegebenen Gebiet, in dem Asphaltmaterial ausgebracht wurde. Um das Asphaltmaterial ausreichend zu verdichten, wird jeder Bereich der Straße mehrmals von einer Straßenwalze überfahren. Dazu fährt die Straßenwalze den Straßenbelag in mehreren Bahnen ab, da dieser üblicherweise breiter ist als die Walzenbandage. Nachdem die Straßenwalze in einzelnen Bahnen den Straßenbelag ein erstes Mal überfahren hat, beginnt diese dann mit einer weiteren Überfahrt bei der ersten bereits überfahrenen Bahn.

Im Stand der Technik gibt es einige Ansätze, wie die Verdichtung bestimmt werden kann: Damit der eingebaute Straßenbelag eine vorgeschriebene Tragfähigkeit erreicht, muss eine gewisse Dichte erreicht werden. Bekannt ist es, die Dichte entweder im Labor anhand des Bohrkerns zu ermitteln oder direkt vor Ort, beispielsweise durch radiometrische Dichtemessungen mittels einer Aufsetzsonde (sogenannte Troxler-Sonde), durchzuführen.

Eine derartige Aufsetzsonde (Handgerät) ist beispielsweise in Heft 7 der Fachzeitschrift "Bitumen" aus dem Jahr 1973 beschrieben (Hintsteiner, E.: "Die zerstörungsfreie Prüfung der Dichte von Asphalt mit Isotopensonden"). Auch das Heft 2 aus dem Jahr 1989 dieser Fachzeitschrift beschäftigt sich u. a. mit Dichtemessungen mittels Aufsetzsonden (Behr, H.: "Schnellprüfverfahren zur Qualitätssicherung im Asphaltstraßenbau").

Auch in der Patentliteratur sind einige Messsysteme bzw. Messvorrichtungen beschrieben: Die WO 03/095984 A2 beschreibt ein System zum Erfassen einer Eigenschaft einer Materialmatte von einem Baufahrzeug. Das System umfasst einen Sensor zum Erfassen der Materialeigenschaft und einen Positionierungsmechanismus, der mit dem Fahrzeug und mit dem Sensor verbunden ist. Der Mechanismus verschiebt den Sensor zwischen ersten und zweiten Positionen in Bezug auf das Fahrzeug, wenn sich das Fahrzeug in Bezug auf die Materialmatte verschiebt.

Weiterhin ist es bekannt, dass Verdichtungswerte der zu verdichtenden Fläche während des Walzens (Verdichtens) gemessen und dem Fahrer auf einem Monitor oder Display angezeigt werden. Beispielsweise sind in der DE 20 2009 008 592 U1 und der DE 694 34 631 T2 Vorrichtungen zu Messen des Verdichtungsgrades einer Bodenfläche erläutert, die einen Monitor oder Display aufweisen, welches in der Fahrerkabine der Verdichtungsmaschine platziert ist und zur Anzeige von gemessenen und/oder berechneten Werten wie beispielsweise Verdichtungswerten dient. Anhand dieser Werte kann der Fahrer dann erkennen, welche Bereiche bereits ausreichend verdichtet wurden und welche noch weiter verdichtet werden müssen.

Die DE 102017107145 beschreibt ein System und Verfahren zur Durchführung einer Verdichtungsoperation. Hier wird ausgehend von einem Zielverdichtungsaufwand eine Steuerung der Verdichtungsoperation durchgeführt. Darüber hinaus sind auch noch die Patentanmeldungen WO 2018/019408 A1, EP 3147406 A1 sowie DE 202009008592 U1 als Stand der Technik zu nennen.

Weiter formt der Artikel mit dem Titel "Intelligent Asphalt Compaction Analyzer - Final Report" Stand der Technik. In Kapitel 4.1 wird ein Verdichtungsanalyzer erläutert, bei welchem Kalibrierdaten eingegeben werden können. Das Ermitteln der Kalibrierdaten ist in Kapitel 4.2.6 erläutert.

Alle Systeme haben nur unter bestimmten Randbedingungen, wie z.B. Umgebungstemperatur oder auch Belagstemperatur eine hinreichend gute Genauigkeit oder sind kompliziert bei der Anwendung. Deshalb besteht der Bedarf nach einem verbesserten Ansatz.

Aufgabe der vorliegenden Erfindung ist es, ein System zu schaffen, dass eine hochgenaue Verdichtungsmessung bei guter Ergonomie ermöglicht.

Die Aufgabe wird durch die unabhängigen Patentansprüche gelöst.

Ausführungsbeispiele der vorliegenden Erfindung schaffen ein System zur Verdichtungsmessung mit einer ersten auf einer Verdichtungsmaschine angeordneten oder anordenbaren Verdichtungsmessvorrichtung, einer zweiten mobilen (z.B. handgetragenen) Verdichtungsmessvorrichtung und einer Berechnungseinheit.

Die ersten Verdichtungsmessvorrichtung ist ausgebildet, um ausgehend von einer gemessenen Beschleunigung einer Messsonde der ersten Messvorrichtung infolge einer Messvibration und/oder einer Beschleunigung eines Verdichtungselements der Verdichtungsmaschine zumindest einen ersten stationären Verdichtungswert zu bestimmen, der Auskunft über eine lokale Verdichtung des Untergrunds oder des Belags für zumindest eine erste Position auf dem zu verdichtenden Untergrund / Belag gibt, und mit zugehöriger Positionsinformation für die erste Position auszugeben. Die zweite Verdichtungsmessvorrichtung ist ausgebildet, um durch Interaktion oder Kontakt mit dem Untergrund oder dem Belag die lokale Verdichtung des Untergrunds oder des Belags für zumindest die erste Position zu ermitteln und ausgehend hiervon zumindest einen ersten mobilen Verdichtungswert für die erste Position auszugeben. Die Berechnungseinheit ist ausgebildet, um den ersten stationären Verdichtungswert und den ersten mobilen Verdichtungswert zu vergleichen, um einen Korrekturwert (z.B. zu Kalibrierungszwecken) zu bestimmen, der von der Abweichung der zwei Verdichtungswerte (erster stationärer Verdichtungswert versus erster mobiler Verdichtungswert) abhängig ist. Alternativ kann auch einfach die Abweichung bestimmt und angezeigt werden.

Die zweite Verdichtungsmessvorrichtung ist handgetragen und weist eine höhere Genauigkeit im Vergleich zu der ersten Verdichtungsmessvorrichtung auf. Ferner umfasst das System eine Positionsbestimmungsvorrichtung oder GNSS-Vorrichtung, die mit der ersten Verdichtungsmessvorrichtung gekoppelt ist, um die Positionsinformation für die erste Position zu ermitteln, wenn die erste Verdichtungsmessvorrichtung an der ersten Position positioniert ist; und eine weitere Positionsbestimmungsvorrichtung oder eine weitere GNSS-Vorrichtung, diemit der zweiten Verdichtungsmessvorrichtung gekoppelt ist, um die Positionsinformation für die erste Position zu ermitteln, wenn die zweite Verdichtungsmessvorrichtung an der ersten Position positioniert ist.

Ausführungsbeispielen der vorliegenden Erfindung liegt die Erkenntnis zugrunde, dass durch die Kombination von zwei unabhängigen Verdichtungsmessvorrichtungen, die beispielsweise auf unterschiedlichen Messprinzipien basieren können, die Genauigkeit der Verdichtungsmessung gesteigert werden kann, wobei gleichzeitig ein hoher Bedienkomfort sichergestellt wird. Beispielsweise wird ein auf der Walze angeordnetes Messsystem zur Aufnahme/Messung von Verdichtungs- bzw. Steifigkeitswerten (die Auskunft über eine vorherrschende Verdichtung oder eine Verdichtungsänderung an der Position der Walze geben) mit einem Handmessgerät, wie z.B. eine Aufsetzsonde, kombiniert. Die Verdichtung bzw. Steifigkeitswerte des ersten Messsystems werden mit den Verdichtungswerten des zweiten Messsystems abgeglichen. Das System auf der Walze kann dann auf Basis der von der Aufsetzsonde (Handgerät) gemessenen bzw. aufgenommen Werte entsprechend re-kalibriert werden. Alternativ bzw. additiv kann es dem Walzenfahrer anhand von korrigierten Werten anzeigen, an welchen Stellen (Position) der Untergrund noch weiter verdichtet werden muss und an welchen Stellen eben nicht.

Ein Vorteil der Erfindung liegt darin, dass gemessene Verdichtungswerte zwischen den beiden Messvorrichtungen schnell und einfach abgeglichen werden können, d. h. - wie oben beschrieben - das System auf der Walze entsprechend re-kalibriert werden kann. Denn in der Regel sind die von der Aufsetzsonde (Handgerät) gemessenen bzw. aufgenommenen Werte sehr genau und auch genauer als die gemessenen bzw. aufgenommenen Werte des Messsystems auf der Walze. Ein Abgleich der Messwerte und anschließende Re-Kalibrierung des Messsystems auf der Walze würde das System in der Folge genauer machen.

Ein weiterer Vorteil der Erfindung ist es, dass auf Basis des korrigierten bzw. re-kalibrierten Systems nun dem Fahrer auf dem Monitor oder Display des Walzenmesssystems gegebenenfalls weitere Stellen (Positionen) auf dem zu verdichtenden Untergrund angezeigt werden, welche noch nicht ausreichend verdichtet sind. Weiterhin werden dem Fahrer gegebenenfalls weitere Stellen (Positionen) auf dem zu verdichtenden Untergrund angezeigt, an welchen bereits genug verdichtet wurde, d. h. keine weitere Verdichtung mehr notwendig ist. Vor einer weiteren Überfahrt über diese Bereiche kann dann die Verdichtungsleistung der Maschine (beispielsweise Vibrationsfrequenz und/oder Amplitude) vermindert werden und so eine etwaige Überverdichtung des Untergrunds vermieden werden. Das vermindert auch den Verschleiß der Maschine (Walze) und reduziert deren Kraftstoffverbrauch, da die in das Material eingebrachte Verdichtungsleistung entsprechend dem bereits gegebenen Verdichtungswert des Untergrunds angepasst wird.

Entsprechend Ausführungsbeispielen ist die erste Verdichtungsmessvorrichtung und/oder die zweite Verdichtungsmessvorrichtung ausgebildet, um z.B. über Funk miteinander zu kommunizieren. Hierzu weist erfindungsgemäß die zweite Verdichtungsmessvorrichtung eine Funkschnittstelle auf, über welche dann die Daten an die Berechnungseinheit übermittelt werden können. Die Berechnungseinheit ist typischerweise (aber nicht notwendigerweise) auf der Walze angeordnet und vergleicht die Messwerte, die sie per Funk von der zweiten Verdichtungsmessvorrichtung (Aufsetzsonde), mit den Messdaten von der ersten Verdichtungsmessvorrichtung, die sie z.B. kabelgebunden / direkt erhält. Dies ermöglicht vorteilhafterweise den Abgleich der Messwerte ohne zusätzliche Mittel. Es sei angemerkt, dass entsprechend Ausführungsbeispielen die Funkschnittstelle, insbesondere die der zweiten Messvorrichtung kann auch dazu genutzt werden, dass die Daten an eine weitere Straßenbaumaschine oder nach Extern, z.B. zu einem Server zur Protokollierung der Messwerte übermittelt werden können. Allgemein können alle Verdichtungswerte in einem Speicher, z.B. einem mit der Berechnungseinheit gekoppelten Speicher zu Protokollierungszwecken gespeichert werden. Deshalb umfasst ein weiteres Ausführungsbeispiel des Systems einen entsprechenden (internen) Speicher. Dieser Speicher dient entsprechend Ausführungsbeispielen dazu, um Verdichtungsmesswerte zwischenzuspeichern, sodass der Abgleich der unterschiedlich ermittelten Verdichtungsmesswerte (mobil/stationär) erfolgen kann, sobald für ein und dieselbe Position die jeweiligen Messwerte vorliegen.

Entsprechend weiteren Ausführungsbeispielen weist jede Messvorrichtung (Aufsetzsonde und Messsystem der Walze) jeweils eine Positionsbestimmungseinheit, wie z.B. GNSSoder GPS-Sensor, auf, wodurch den Messwerten Positionsdaten zugeordnet werden können, um die mobilen und stationären Messwerte anhand der Positionsdaten zueinander zuzuordnen bzw. auseinanderhalten zu können. Hintergrund ist, dass typischerweise die Messung nicht gleichzeitig erfolgt, sondern immer dann, wenn das jeweilige Messgerät (Messvorrichtung der Walze bzw. das Handmessgerät) an der jeweiligen Position aufgesetzt bzw. positioniert ist. Folglich ist die positionstechnische Zuordnung vorteilhaft, um die genaue Abweichung für eine Messung mit den zwei unterschiedlichen Messsystemen an derselben Position bestimmen zu können.

Entsprechend Ausführungsbeispielen wird mittels der Vorrichtung dieser Abgleich mehrfach durchgeführt, so dass also mit der ersten Verdichtungsmessvorrichtung ein zweiter stationärer Verdichtungswert für eine zweite Position ermittelt und ausgegeben wird, während mit der zweiten Verdichtungsmessvorrichtung ein zweiter mobiler Verdichtungswert für die zweite Position bestimmt wird. An dieser Stelle sein angemerkt, dass die Verdichtungswerte (erster und zweiter) typischerweise beabstandet voneinander sind, da die mobilen Verdichtungswerte nicht zur kontinuierlichen Überwachung dienen, sondern vor allem zur Kalibrierung bzw. zum Überprüfen stichprobenartig eingesetzt werden. Im Gegensatz dazu kann entsprechend Ausführungsbeispielen zumindest die erste Verdichtungsmessvorrichtung kontinuierliche Messwerte erfassen, d. h. stationäre Verdichtungswerte über einen zeitlichen Verlauf, derart, dass die stationären Verdichtungsmesswerte einem zeitlichen Verlauf einer Bewegungsbahn der Verdichtungsmaschine oder mehreren Positionen der Bewegungsbahn der Verdichtungsmaschine zuordenbar sind.

Entsprechend Ausführungsbeispielen ist die zweite Verdichtungsmessvorrichtung als Troxler-Sonde oder als vergleichbare Vorrichtung ausgeführt, die einen Gammastrahler und/oder einen Neutronenstrahler in Kombination mit einem Zählrohr zur Zählung der zurückreflektierten Strahlung umfasst. Derartige Troxler-Sonden ermöglichen vorteilhafterweise eine hochgenaue Messung der Dichte und damit der Verdichtung. Alternativ kann ein Aufsetzsensor, der mit einem elektromagnetischen Signal im Bereich von 1Mhz arbeitet verwendet werden. Diese Aufsetzsensor (zweite Verdichtungsmessvorrichtung) umfasst z.B. einen Megahertz-Sender, eine Megahertz-Empfänger und einen Prozessor. Der Megahertz-Sender ist ausgebildet, um ein elektromagnetisches Feld bei etwa 1 MHz oder im Bereich 0,3 MHz bis 5 MHz oder 0,5 MHz bis 2,5 MHz (allgemein größer 0,1 oder 0,3 oder 0,5 MHz bereitzustellen; der Megahertz-Empfänger empfangt ein aus dem elektromagnetischen Feld resultierendes Antwortsignal, wobei der Prozessor das Antwortsignal auswertet, um den ersten mobilen Verdichtungswert zu ermitteln. Entsprechend weiteren Ausführungsbeispielen kann alternativ als zweite Verdichtungsmessvorrichtung zur Ermittlung der mobilen Verdichtungswerte auch eine weitere Verdichtungsmessvorrichtung, die auf einer weiteren Straßenbaumaschine, wie z.B. einer weiteren Walze positioniert ist, verwendet werden. Diese Verdichtungsmesswerte werden beispielsweise mittels Funk die an die Berechnungseinheit, z.B. die Berechnungseinheit, die auf der Walze mit der ersten Verdichtungsmessvorrichtung angeordnet ist, übermittelt.

Entsprechend einem weiteren Ausführungsbeispiel kann das System auch noch einen Temperatursensor umfassen, der ausgebildet ist, um die Temperatur des Untergrunds oder des Belags zumindest an der ersten Position zu ermitteln und einen Betriebstemperaturwert zugeordnet zu der Positionsinformation für die erste Position auszugeben. Dies ist deshalb vorteilhaft, weil die Verdichtungswerte, insbesondere die Änderung der Verdichtungswerte im Zusammenhang mit der Temperatur zu sehen sind, um die Güte der aufgebrachten Schicht zu beurteilen. Deshalb kann entsprechend Ausführungsbeispielen auch der Temperaturwert entsprechend mitprotokolliert werden.

Ein weiteres Ausführungsbeispiel bezieht sich auf eine Baumaschine, wie z.B. eine Verdichtungsmaschine oder Walze umfassend das oben charakterisierte System.

Ein weiteres Ausführungsbeispiel bezieht sich auf ein Verfahren zur Verdichtungsmessung mit den Schritten:
- Ermitteln zumindest eines ersten stationären Verdichtungswerts zusammen mit einer Positionsinformation für eine erste Position mittels einer ersten Verdichtungsmessvorrichtung, die auf einer Verdichtungsmaschine angeordnet ist, unter Verwendung einer gemessenen Beschleunigung einer Messsonde infolge einer Messvibration und/oder einer Beschleunigung eines Verdichtungselements der Verdichtungsmaschine, wobei der zumindest erste stationäre Verdichtungswert eine lokale Verdichtung des Untergrunds für die erste Position anzeigt;
- Ermitteln zumindest eines ersten mobilen Verdichtungswerts mit zugehöriger Positionsinformation für die erste Position mittels einer zweiten Verdichtungsmessvorrichtung, die mobil ist, bei Interaktion oder Kontakt mit dem Untergrund; und
- Vergleichen des ersten stationären Verdichtungswerts und des ersten mobilen Verdichtungswerts, um einen Korrekturwert zu bestimmen, der von der Abweichung des ersten stationären Verdichtungswerts von dem ersten mobilen Verdichtungswert abhängig ist, oder um eine Abweichung zu bestimmen und anzuzeigen.

Dieses Verfahren kann selbstverständlich auch Computer-implementiert realisiert sein.

Weiterbildungen sind in den Unteransprüchen definiert. Ausführungsbeispiele werden nachfolgend anhand der beiliegenden Zeichnungen erläutert. Es zeigen:
Fig. 1 eine schematische Darstellung eines Systems gemäß einem Basisausführungsbeispiel; und
Fig. 2 eine schematische Darstellung einer Verdichtungsvorrichtung mit einem entsprechenden Messsystem gemäß einem erweiterten Ausführungsbeispiel;

Bevor nachfolgend Ausführungsbeispiele der vorliegenden Erfindung anhand der beiliegenden Zeichnungen erläutert werden, sei darauf hingewiesen, dass gleichwirkende Elemente und Strukturen mit gleichen Bezugszeichen versehen sind, so dass die Beschreibung derer aufeinander anwendbar bzw. austauschbar ist.

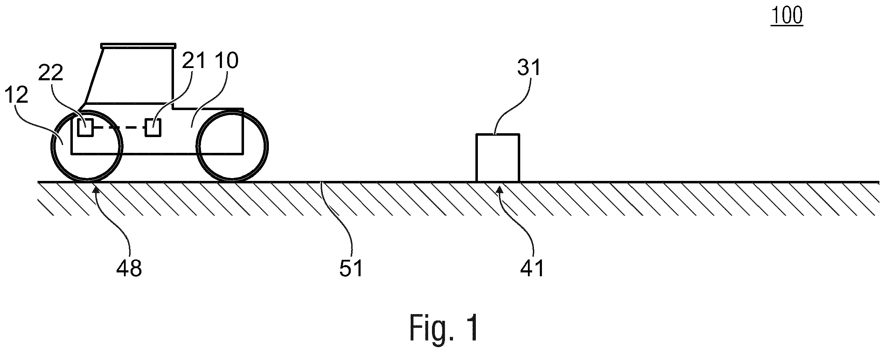

Fig. 1 zeigt ein System 100 umfassend einen ersten Verdichtungssensor 22 sowie einen zweiten Verdichtungssensor 31. Darüber hinaus umfasst das System 100 auch noch eine Berechnungseinheit 21.

Der erste Verdichtungssensor 22 ist z.B. auf einer Baumaschine, hier einer Walze 10 anordenbar bzw. angeordnet und ausgebildet, um während der Verdichtung des Untergrunds 51 eine lokale Verdichtung an der Messposition 48 (Standposition der Walze 10 bzw. Überfahrposition der Walze 10) zu ermitteln. Hierzu ermittelt beispielsweise die erste Verdichtungsmessvorrichtung 22 ausgehend von einer mittels einer Messsonde gemessenen Beschleunigung, die aus der Vibration des Verdichtungselements 12 der Walze 10 herrührt, einem ersten stationären Verdichtungswert, der Auskunft über die lokale Verdichtung einer Position 48 gibt. Der Hintergrund hierzu ist, dass die mittels der Messsonde der Verdichtungsmessvorrichtung 22 ermittelte Beschleunigung im Verhältnis zu der Anregung eine Auskunft über die Dämpfung durch den Untergrund 51 einer lokalen Position 48 gibt. Diese Dämpfung ist abhängig von der Verdichtung bzw. insbesondere der Verdichtungsänderung beim Einbringen der Vibration mittels Verdichtungselements 12. Bezüglich des genauen Messprinzips sei beispielsweise auf die EP 3 147 406 A1 verwiesen. Diese Schrift beschreibt, dass die Bestimmung des Verdichtungsgrads bei jeder Überfahrt der Walze über die zu verdichtende Fläche dadurch erfolgt, dass die in den Untergrund eingebrachte bzw. die in dem Untergrund wirkenden Kräfte mittels eines Verdichtungsmessers 22 gemessen werden. Aus den gemessenen Werten kann dann ein Grad der Verdichtung des Untergrunds berechnet werden. Im Falle des mehrmaligen Überfahrens über die zu verdichtende Fläche kann auch eine Änderung der Verdichtung des Untergrunds ermittelt werden. Alternativ zu diesem beschriebenen System kann das Verdichtungsmesssystem nach der DE 10 2010 054 755 A1 verwendet werden. Dieses umfasst mindestens zwei Systeme zur Bestimmung des Verdichtungszustands verschiedener Teile eines Materialbereichs auf einer Baustelle sowie ein Anzeigesystem zur Darstellung gesammelter Daten. Alle diese Systeme sind dazu ausgebildet, um einen Verdichtungswert, der nachfolgend als stationärer Verdichtungswert bezeichnet wird, zu ermitteln. An der Position 48 ist dieser stationäre Verdichtungswert der erste stationäre Verdichtungswert.

Die Verdichtungsmessvorrichtung 31 ist mobil und ausgebildet, bei Interaktion oder Kontakt mit dem Untergrund 51 die lokale Verdichtung an dieser Position (vgl. Position 41) zu bestimmen. Wenn die Sonde 31 an der Position 41 aufgesetzt wird, kann mittels dieser ein erster mobiler Verdichtungswert bestimmt werden. Als mobile Verdichtungsmessvorrichtung 31 eignet sich beispielsweise eine Troxler-Sonde, die z.B. in der US 9,618,496 B2 beschrieben ist. Alternative kann auch ein andersartiger Aufsetzsensor, z.B. auf MHz-Basis verwendet werden. Hierbei handelt es sich um eine Aufsetzsonde bzw. allgemein um eine Messvorrichtung, die zum Messen einer Eigenschaft eines mit dem Straßenbelag in Beziehung stehenden Materials konfiguriert ist. Derartige Vorrichtungen sind zwar nicht dazu geeignet, kontinuierlich (vgl. Bereich von 41 bis 48) Messwerte zu erfassen, sondern nur wenn sie aufgesetzt werden, zeichnen sich allerdings durch erhöhte Genauigkeit bei der Verdichtungsgradbestimmung aus. Diese erhöhte Genauigkeit kann man sich entsprechend dem hier offenbarten Konzept dadurch zunutze machen, dass ein an der Stelle 41 ermittelter erster mobiler Verdichtungswert mit dem ersten stationären Verdichtungswert abgeglichen wird, um beispielsweise Kalibrierdaten oder auch nur die Abweichung zwischen den zwei Verdichtungswerten zu bestimmen. Dieser Abgleich wird durch die Berechnungseinheit 21 durchgeführt, die beispielsweise mit der Verdichtungsmessvorrichtung 22 gekoppelt ist oder beispielsweise auf der Walze 10 angeordnet ist. An dieser Stelle sei angemerkt, dass die Anordnung auf der Walze nicht zwingend ist, sondern dass auch diese Vorrichtung extern vorgesehen sein kann. Der Verdichtungssensor 31 übermittelt seine Messwerte (vgl. erster mobiler Verdichtungswert) an die Berechnungseinheit 21, z. B. unter Zuhilfenahme einer Funkschnittstelle (z.B. Bluetooth oder WLAN). Analog hierzu übermittelt die Messvorrichtung 22 die stationären Messwerte (vgl. erster stationärer Messwert) ebenfalls an die Berechnungseinheit 21, z. B. per Kabelverbindung oder per Funkverbindung. Diese Messwerte werden dann miteinander verglichen, um dann beispielsweise Kalibrierdaten zu bestimmen.

Entsprechend Ausführungsbeispielen können beide Messvorrichtungen 22 und 31 jeweils mit einer Positionsbestimmungsvorrichtung, wie z. B. einem GPS-Sensor oder einem GNSS-Sensor kombiniert sein, so dass direkt zu den Verdichtungswerten eine Positionsinformation vorliegt, was den Abgleich wesentlich erleichtert, da er nicht gleichzeitig an der ersten Position 48 gemessen wird. Diese Positionsinformation wird dem jeweiligen Messwert zugeordnet und dann durch die Berechnungseinheit 21 verarbeitet. Entsprechend Ausführungsbeispielen kann diese Verarbeitung allerdings auch derart erfolgen, dass sie Messwerte beispielsweise manuell an Positionsinformationen derart, dass eine erste Kalibrierposition blockiert wird, zugeordnet ist. Hierzu sind dann keine genauen Positionsdaten notwendig.

Nachfolgend wird das bei diesem Ansatz bzw. diesem System zugrunde liegende Verfahren erläutert:
- Messsystem auf der Walze ermittelt beim Überfahren des zu verdichtenden Untergrunds Verdichtungswerte sowie dazugehörige Positionsangaben;
- Gemessene Verdichtungswerte werden zusammen mit den dazugehörigen Positionsangaben drahtlos an eine Aufsetzsonde (mobiles Handgerät) gesendet;
- Mit der Aufsetzsonde (mobiles Handgerät) werden anschließend an den entsprechenden Positionen des bereits verdichtenden Untergrunds Verdichtungswerte nachgemessen und mit denen durch das Messsystem auf der Walze ermittelten Werten verglichen;
- Durch den Vergleich der Messwerte wird auf dem Handgerät (Aufsetzsonde) angezeigt, inwieweit die Messwerte voneinander abweichen, d. h. ob eine Verdichtung des Untergrunds an den entsprechenden Positionen in Ordnung ist oder ob der Untergrund noch weiter verdichtet werden muss;
- Gemessene Verdichtungswerte durch das Handgerät (Aufsetzsonde) werden zusammen mit den dazugehörigen Positionsangaben drahtlos an das Messsystem auf der Walze gesendet;
- Messsystem auf der Walze führt (a) auf Basis der Messwerte des Handgeräts (Aufsetzsonde) eine Re-Kalibrierung bzw. Korrektur aus, um das System in der Folge genauer zu machen, und zeigt (b) dem Fahrer an, an welchen Stellen (Positionen) der Untergrund noch weiter verdichtet werden muss oder keine Verdichtung mehr notwendig ist.

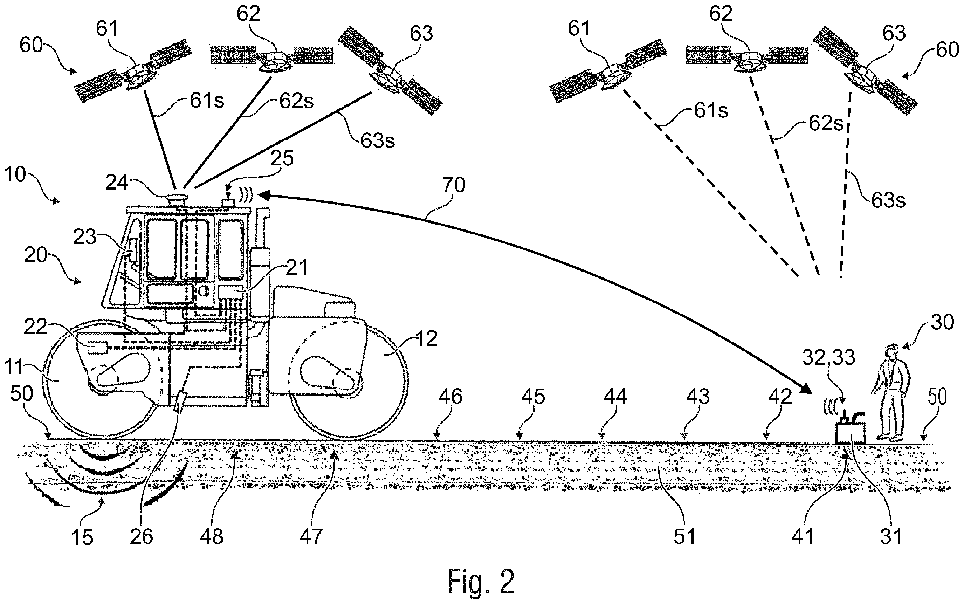

In Fig. 2 ist eine Walze 10 mit einer vibrierenden Bandage 11 und einer nicht vibrierenden Bandage 12 dargestellt, welche sich auf der Oberfläche 50 eines zu verdichtenden Untergrundes 51 bewegt. Durch die Bandage 11 werden entsprechende Vibrationen 15 in den Untergrund 51 eingebracht, d. h. der Untergrund 51 wird durch die Bandage 11 zusätzlich verdichtet. Die Walze 10 weist weiterhin eine Vorrichtung zur Regelung der Verdichtungsleistung (in der Figur nicht dargestellt) sowie ein Messsystem 20 zum Bestimmen eines Verdichtungswertes des Untergrunds 51 bzw. der Materialschicht 51 auf. Das Messsystem 20 besteht im Wesentlichen aus einem im Bereich der vorderen Bandage 11 und dem Chassis der Walze 10 angeordneten Schwingungsaufnehmer 22 (beispielsweise ein Beschleunigungssensor), aus einer auf dem Dach der Walze 10 angeordneten Positionsbestimmungseinheit 24 (GNSS / GPS), aus einer ebenfalls auf dem Dach der Walze 10 angeordneten Kommunikationseinheit 25 zum Senden und Empfangen von insbesondere Mess- und Positionsdaten, aus einer Berechnungseinheit 21, die die Positionsauswertung und die Messwertauswertung beherbergt, sowie einer im Fahrerstand angeordneten Anzeige- und Bedieneinheit 23. Zusätzlich kann das Messsystem 20 einen im unteren Bereich und vorzugsweise zwischen den beiden Bandagen 11 und 12 angeordneten berührungslosen Temperatursensor 26 aufweisen. Auch kann eine optionale Verbindungsbox (hier nicht dargestellt), in welcher alle genannten Komponenten des Verdichtungsmesssystems 20 mittels elektrischer Verbinder angeschlossen sind, vorgesehen sein.

Die auf dem Dach der Walze 10 angeordnete Positionsbestimmungseinheit 24 (GNSS / GPS) empfängt Satellitensignale 61s, 62s, 63s von einem Satellitensystem 60, welches in Fig. 2 durch drei Satelliten 61 bis 63 dargestellt ist. Anhand der Satellitensignale 61s, 62s, 63s kann die genaue Position der Walze 10 kontinuierlich ermittelt werden. Somit ist es möglich, den mittels des Messsystems 20 aufgenommenen Verdichtungsmesswerten eine genaue Positionsangabe hinzuzufügen, d.h. jedem aufgenommenen Messwert kann eine Position auf der Fläche 50 bzw. auf dem Untergrund 51 zugeordnet werden und dann beispielsweise dem Walzenfahrer grafisch auf der Anzeige- und Bedieneinheit 23 visualisiert werden. Auch können Positionsdaten zusammen mit den Verdichtungswerten in der Berechnungseinheit 21 für eine spätere Weiterverarbeitung oder zu Protokollzwecken abgespeichert werden. Bei einer Verdichtung durch die Walze 10, d.h. während der Überfahrt der Walze 10 über die zu verdichtende Fläche 50 des Untergrunds 51, werden Beschleunigungen mittels des Schwingungsaufnehmers 22 gemessen und in der Berechnungseinheit 21 entsprechend ausgewertet. Der GNSS-Empfänger 24 liefert die nötigen Positionsinformationen an die Berechnungseinheit 21. Zusätzlich können zu der Ermittlung und Auswertung der reinen Beschleunigung auch noch zusätzliche Parameter, wie z.B. die Temperatur des Planums (ermittelt mittels des Temperatursensors 26) mitberücksichtigt werden.

In Bezug auf die Ausführungen des Messsystems 20 sei darauf hingewiesen, dass die einzelne Implementierung des dargestellten Systems variieren kann. So kann beispielsweise bei der Implementierung der Verdichtungssensor 22, welcher an dem Lagergestell im Bereich der Bandage 11 angeordnet ist, auch woanders im Lagergestell bzw. im Lager selbst oder im Bereich der zweiten Bandage 12 angeordnet sein. Alternativ hierzu wäre es auch möglich, dass zwei derartige Verdichtungssensoren für die zwei Bandagen 11 und 12 vorgesehen sind. Im Allgemeinen heißt das, dass auch mehrere Schwingungssensoren (bspw. zwei (an den Bandagen vorne und hinten) oder vier (zwei an der Bandage vorne links/rechts; zwei an der Bandage hinten links/rechts) an der Walzeneinrichtung angeordnet sein können.

Wie in Fig. 2 dargestellt, so bewegt sich die Walze 10 während des Verdichtungsvorgangs über eine Oberfläche 50 des Untergrunds 51. Einzelne Messstellen auf der zu verdichtenden Fläche 50 bzw. des zu verdichtenden Untergrunds 51, die von der Walze 10 während des Verdichtungsvorgangs überfahren werden, sind mit den Bezugszeichen 41 bis 48 gekennzeichnet. An diesen (meist vorher festgelegten) Messstellen 41 bis 48 werden zusätzliche Verdichtungsmessungen durch eine Person 30 durchgeführt, und zwar mit einem Handgerät 31, beispielsweise einem radiometrischen Dichtemessgerät (Aufsetzsonde, sogenannte Troxler-Sonde). Die zusätzlichen Verdichtungsmessungen durch die Person 30 dienen in der Regel zur Überprüfung und Protokollierung der durch die Walze 10 durchgeführten Verdichtung. Solche Überprüfungsmessungen sind als sogenannte Qualitätssicherungsmaßnahme teilweise vorgeschrieben, d.h. vom Auftraggeber vorgegeben.

Das Handgerät 31 verfügt über eine Positionsbestimmungseinheit 33 (GNSS / GPS), beispielsweise mit einem im Gehäuse des Handgeräts 31 integrierten Empfänger, sowie über eine Kommunikationseinheit 32 zum Senden und Empfangen von insbesondere Mess- und Positionsdaten. Die am oder im Handgerät 31 angeordnete Positionsbestimmungseinheit 33 (GNSS / GPS) empfängt ebenfalls, d.h. wie auch das Messsystem 20 auf der Walze 10, Satellitensignale 61s, 62s, 63s von dem Satellitensystem 60, dargestellt in Fig. 2 durch drei Satelliten 61 bis 63. Anhand der Satellitensignale 61s, 62s, 63s kann das Handgeräts 31 die genaue Position ermitteln. Somit ist es möglich, den mittels des Handgeräts 31 aufgenommenen Verdichtungsmesswerten eine genaue Positionsangabe (GNSS- /GPS-Koordinaten) hinzuzufügen, d.h. jedem an den Messstellen 41 bis 48 aufgenommenen Messwert kann eine Position auf der Fläche 50 bzw. auf dem Untergrund 51 zugeordnet werden. Auch können Positionsdaten zusammen mit den Verdichtungswerten in dem Handgerät 31 für eine spätere Weiterverarbeitung oder zu Protokollzwecken abgespeichert werden. Wie in Fig. 2 dargestellt, so führt die Person 30 eine Verdichtungsmessung der bereits durch die Walze 10 verdichtenden Fläche 50 bzw. des bereits verdichtenden Untergrunds 51 mit dem Handgerät 31 an der Messstelle 41 durch. Der erfindungsgemäße Gedanke besteht nun darin, dass die von dem auf der Walze 10 angeordneten Messsystem 20 aufgenommenen (gemessenen) Verdichtungs- bzw. Steifigkeitswerte mit denen durch die Aufsetzsonde (Handgerät 31) aufgenommen (gemessenen) Verdichtungswerte abgeglichen werden. Dazu sendet das Messsystem 20 auf der Walze 10 die beim Überfahren der zu verdichtenden Fläche 50 bzw. des zu verdichtenden Untergrunds 51 ermittelten Verdichtungswerte sowie die dazugehörigen Positionsangaben mittels der auf dem Dach der Walze 10 angeordneten Kommunikationseinheit 25 drahtlos an das mobile Handgerät 31.

Eine Person 30 misst anschließend mit dem mobilen Handgerät 31 an den entsprechenden Messstellen 41 bis 48 des bereits verdichtenden Untergrunds 51 eine dort vorhandene Verdichtung nach. Im Handgerät 31 werden die ermittelten Werte mit denen durch das Messsystem 20 auf der Walze 10 ermittelten Werte verglichen und es wird direkt für die Person 30 ersichtlich auf dem Handgerät 31 angezeigt, inwieweit die Messwerte voneinander abweichen, d. h. ob eine Verdichtung des Untergrunds 51 an den entsprechenden Messstellen 41 bis 48 in Ordnung ist oder ob der Untergrund 51 noch weiter verdichtet werden muss. Die durch das Handgerät 31 gemessenen Verdichtungswerte werden anschließend zusammen mit den dazugehörigen Positionsangaben mittels der in oder am Handgerät 31 angeordneten Kommunikationseinheit 32 drahtlos an das Messsystem 20 auf der Walze 10 gesendet. Eine bidirektionale Datenkommunikationsverbindung zwischen Handgerät 31 und dem Messsystem 20 auf der Walze 10 ist in Fig. 2 dargestellt durch einen Doppelpfeil 70. Denkbar ist in diesem Zusammenhang auch, dass diese Daten auch von anderen (weiteren) Walzen auf der Baustelle empfangen werden. Weiterhin denkbar ist, dass die Daten entweder direkt per drahtloser Kommunikationsverbindung zwischen Handgerät 31 und Walze(n) ausgetauscht werden, oder indirekt durch "Ablage" auf einem Server (das Handgerät 31 könnte die Daten auch auf einem zentralen Server ablegen).

Das Messsystem 20 auf der Walze 10 führt nach Erhalt der vom Handgerät 31 gemessenen Verdichtungs- und Positionswerte eine Re-Kalibrierung bzw. Korrektur auf Basis dieser Werte durch. Die korrigierten Messwerte werden dem Walzenfahrer auch auf der Anzeige- und Bedieneinheit 23 dargestellt, damit dieser sofort ersehen kann, an welchen Stellen (Messstellen 41 bis 48) der Untergrund 51 noch weiter verdichtet werden muss oder keine Verdichtung mehr notwendig ist.

Wie oben angegeben, so kann an der Walze 10 auch ein Temperatursensor 26 angeordnet sein. Insbesondere beim Walzen von Asphaltmaterial können so bei jeder Überfahrt Temperaturdaten mit aufgenommen werden und beispielsweise ein Abkühlverhalten des Asphaltmaterials errechnet werden. Die Temperaturdaten und das Abkühlverhalten des Asphalts können bei einer Re-Kalibrierung bzw. Korrektur des Messsystems 20 auf der Walze 10 auf Basis der Messwerte des Handgeräts 31 (Aufsetzsonde) mitberücksichtigt werden

Entsprechend weiteren Ausführungsbeispielen können die gemessenen Verdichtungswerte durch das Handgerät (Aufsetzsonde), welche zusammen mit den dazugehörigen Positionsangaben drahtlos an das Messsystem auf der Walze gesendet werden, auch von anderen Walzen verwendet werden (im Straßenbau sind meist mehrere Walzen im Einsatz). Die Daten werden von den anderen Walzen entweder direkt per drahtloser Kommunikationsverbindung empfangen oder indirekt durch "Ablage" auf einem Server (das Handgerät könnte die Daten auch auf einem zentralen Server ablegen).

Auch wenn bei obigen Ausführungsbeispielen davon ausgegangen wurde, dass das zweite Verdichtungsmessgerät ein Handgerät ist, so kann alternativ als Handgerät (Aufsetzsonde) auch ein an einer weiteren Walze angeordnetes Gerät verwendet werden, wie bspw. in der eingangs genannten WO 03/095984 A2 beschrieben. Die Walze fährt die von dem Messsystem der anderen Walze ermittelten und per drahtloser Kommunikationsvorrichtung empfangenen Positionen an, um dort Verdichtungswerte nachzumessen und mit denen durch das Messsystem auf der anderen Walze ermittelten Werten zu vergleichen. Anschließend werden die gemessenen Verdichtungswerte zusammen mit den dazugehörigen Positionsangaben drahtlos an das Messsystem auf der anderen Walze gesendet.

Bezugnehmend auf Fig. 1 sei erläutert, dass, wie im Zusammenhang mit Fig. 2 erläutert, auch ein zusätzlicher Temperatursensor an der Walze für die Asphaltverdichtung eingesetzt werden kann. Bei den Überfahrten der Walze über einen zu verdichtenden Asphalt-Straßenbelag werden Temperaturdaten mit aufgenommen und so durch das Messsystem auf der Walze ein Abkühlverhalten errechnet (Asphalt kühlt nach dem Aufbringen durch den Straßenfertiger kontinuierlich aus). Die Temperaturdaten und das Abkühlverhalten des Asphalts werden bei einer Re-Kalibrierung bzw. Korrektur des Messsystems auf der Walze auf Basis der Messwerte des Handgeräts (Aufsetzsonde) mit berücksichtigt. Beispielsweise gehen die Temperaturdaten und das Abkühlverhalten des Asphalts mit in eine einzustellende Verdichtungsleistung der Walze ein. Insofern muss in Abhängigkeit des Temperaturabfalls eine Verdichtungsleistung der Maschine mehr oder minder stark zurückgenommen werden.

Entsprechend weiteren Ausführungsbeispielen kann statt der Temperaturmessvorrichtung auch eine Feuchtigkeitsmessvorrichtung verwendet werden, die insbesondere dann relevant ist, wenn nicht eine Asphaltdecke verdichtet werden soll, sondern ein loser Untergrund, wie z. B. Schotter. Hierbei ist die Verdichtung im Regelfall im Zusammenhang mit einem Feuchtigkeitsgrad des zu verdichtenden Materials zu sehen. Deshalb umfasst ein zusätzliches Ausführungsbeispiel einen Feuchtigkeitssensor an der Verdichtungsvorrichtung.

Wie eingangs erläutert, dient die Ermittlung der Verdichtungswerte insbesondere zur Protokollierung. Deshalb werden vorzugsweise alle aufgenommenen bzw. ermittelten Verdichtungswerte (zusammen mit den entsprechenden Positionsangaben) protokolliert, um im Nachhinein die durchgeführten Verdichtungsarbeiten auswerten zu können (beispielsweise ein Nachweis für den Auftraggeber).

An dieser Stelle darauf hingewiesen, dass Ausführungsbeispiele der vorliegenden Erfindung sowohl im Straßenbau (Verdichtung von Asphaltmaterial) als auch im Erdbau einsetzbar sind, d. h. also beim Verdichten von Erde, Schotter oder aber auch sandigen Untergründen Verwendung finden.

Zur Positionsbestimmungsvorrichtung 24 / 33 (GPS/GNSS) sei angemerkt, dass entsprechend weiteren Ausführungsbeispielen auch Korrektursignal für das GNSS-Signal verwendet werden kann, z. B. ein Korrektursignal von einem stationären Sender oder einem geostationären Sender. Dieses Korrektursignal bzw. Zusatzsignal erhöht die Genauigkeit einer Positionsbestimmung signifikant.

Die oben beschriebenen Ausführungsbeispiele stellen lediglich eine Veranschaulichung der Prinzipien der vorliegenden Erfindung dar. Es versteht sich, dass Modifikationen und Variationen der hierin beschriebenen Anordnungen und Einzelheiten anderen Fachleuten einleuchten werden. Deshalb ist beabsichtigt, dass die Erfindung lediglich durch den Schutzumfang der nachstehenden Patentansprüche und nicht durch die spezifischen Einzelheiten, die anhand der Beschreibung und der Erläuterung der Ausführungsbeispiele hierin präsentiert wurden, beschränkt sei.

## Patentansprüche

1. System (100) zur Verdichtungsmessung mit folgenden Merkmalen:
einer ersten Verdichtungsmessvorrichtung (22), die auf einer Verdichtungsmaschine (10) anordenbar ist und ausgebildet ist, um ausgehend von einer gemessenen Beschleunigung einer Messsonde der ersten Verdichtungsmessvorrichtung (22) infolge einer Messvibration (15) und/oder einer Beschleunigung eines Verdichtungselements (11, 12) der Verdichtungsmaschine (10) zumindest einen ersten stationären Verdichtungswert zu bestimmen, der eine lokale Verdichtung des Untergrunds (51) oder des Belags für zumindest eine erste Position (48) anzeigt, und mit zugehöriger Positionsinformation für die erste Position (48) auszugeben;
einer zweiten Verdichtungsmessvorrichtung (31), die mobil ist und ausgebildet ist, um bei Interaktion oder Kontakt mit dem Untergrund (51) oder dem Belag die lokale Verdichtung des Untergrunds (51) oder des Belags für zumindest die erste Position (48) zu ermitteln und ausgehend hiervon zumindest einen ersten mobilen Verdichtungswert mit zugehöriger Positionsinformation für die erste Position (48) auszugeben; wobei die zweite Verdichtungsmessvorrichtung (31) handgetragenen ist; wobei die zweite Verdichtungsmessvorrichtung (31) eine höhere Genauigkeit hat als die erste Verdichtungsmessvorrichtung (22);
eine Berechnungseinheit (21), die ausgebildet ist, um den ersten stationären Verdichtungswert und den ersten mobilen Verdichtungswert zu vergleichen, um einen Korrekturwert für die erste Verdichtungsmessvorrichtung (22) zu bestimmen, der von der Abweichung des ersten stationären Verdichtungswerts gegenüber dem ersten mobilen Verdichtungswert abhängig ist;
eine Positionsbestimmungsvorrichtung (24, 33) oder GNSS-Vorrichtung, die mit der ersten Verdichtungsmessvorrichtung (22) gekoppelt ist, um die Positionsinformation für die erste Position (48) zu ermitteln, wenn die ersten Verdichtungsmessvorrichtung (22) an der ersten Position (48) positioniert ist; und eine weitere Positionsbestimmungsvorrichtung (24, 33) oder eine weitere GNSS-Vorrichtung, , um die Positionsinformation für die erste Position (48) zu ermitteln, wenn die zweite Verdichtungsmessvorrichtung (31) an der ersten Position (48) positioniert ist;
wobei die zweite Verdichtungsmessvorrichtung (31) die weitere Positionsbestimmungsvorrichtung (24, 33) oder die weitere GNSS-Vorrichtung aufweist sowie eine Funkschnittstelle (31) umfasst, die ausgebildet ist, um zumindest den ersten mobilen Verdichtungswert an die Berechnungseinheit (21) zu übermitteln.

2. System (100) gemäß Anspruch 1, wobei die erste Verdichtungsmessvorrichtung (22) ausgebildet ist, um einen zweiten stationären Verdichtungswert für eine zweite Position (48) zu ermitteln und auszugeben; und wobei die zweite Verdichtungsmessvorrichtung (31) ausgebildet ist, um einen zweiten mobilen Verdichtungswert für die zweite Position (48) zu bestimmen und auszugeben; und/oder
wobei die Berechnungseinheit (21) ausgebildet ist, um den ersten stationären Verdichtungswert und den ersten mobilen Verdichtungswert anhand der Positionsinformationen einander zuzuordnen und zu vergleichen und um den zweiten stationären Verdichtungswert und den zweiten mobilen Verdichtungswert anhand der Positionsinformationen einander zuzuordnen und zu vergleichen.

3. System (100) gemäß einem der vorherigen Ansprüche, wobei die erste Verdichtungsmessvorrichtung (22) ausgebildet ist, um stationäre Verdichtungswerte über einen zeitlichen Verlauf zu bestimmen und die stationären Verdichtungswerte einem zeitlichen Verlauf einer Bewegungsbahn der Verdichtungsmaschine (10) und/oder mehrere Positionen (48) der Bewegungsbahn der Verdichtungsmaschine (10) zuzuordnen.

4. System (100) gemäß einem der vorherigen Ansprüche, wobei die zweite Verdichtungsmessvorrichtung (31) ausgebildet ist, um zumindest den ersten mobilen Verdichtungswert zu ermitteln, wenn die zweite Verdichtungsmessvorrichtung (31) auf die erste Position (48) aufgesetzt wird; und/oder
wobei die zweite Verdichtungsmessvorrichtung (31) ausgebildet ist, um zumindest einen zweiten mobilen Verdichtungswert für eine zweite von der ersten Position (48) beabstandeten Position (48) zu ermitteln, wenn die zweite Verdichtungsmessvorrichtung (31) auf die zweite Position (48) aufgesetzt wird.

5. System (100) gemäß einem der vorherigen Ansprüche, wobei die zweite Verdichtungsmessvorrichtung (31) eine Troxlersonde umfasst; und/oder
wobei die zweite Verdichtungsmessvorrichtung (31) einen Gammastrahler und/oder Neutronenstrahler in Kombination mit einem Zählrohr umfasst; oder
wobei die zweite Verdichtungsmessvorrichtung (31) einen Megahertz-Sender, eine Megahertz-Empfänger und einen Prozessor umfasst, wobei der Megahertz-Sender ausgebildet ist, um ein elektromagnetisches Feld bei 1 MHz oder im Bereich größer 0,3 MHz bereitzustellen, wobei der Megahertz-Empfänger ausgebildet ist, um ein aus dem elektromagnetischen Feld resultierendes Antwortsignal zu empfangen, und wobei der Prozessor ausgebildet ist, um das Antwortsignal auszuwerten, um den ersten mobilen Verdichtungswert zu ermitteln.

6. System (100) gemäß einem der vorherigen Ansprüche, wobei die erste Verdichtungsmessvorrichtung (22) eine Schnittstelle umfasst, die ausgebildet ist, um zumindest den ersten stationären Verdichtungswert an die Berechnungseinheit (21) zu übermitteln, und/oder wobei die Berechnungseinheit (21) mit der ersten Verdichtungsmessvorrichtung (22) gekoppelt ist.

7. System (100) gemäß einem der vorherigen Ansprüche, wobei die Funkschnittstelle (31) ausgebildet ist, um zumindest den ersten mobilen Verdichtungswert auch an eine weitere Baumaschine oder an einen Server zu übermitteln.

8. System (100) gemäß einem der vorherigen Ansprüche, wobei das System (100) einen Temperatursensor (26) umfasst, der ausgebildet ist, um die Temperatur des Untergrunds (51) oder des Belags zumindest an der ersten Position (48) zu ermitteln und einen ermittelten Temperaturwert zugehörig zu der Positionsinformation für die erste Position (48) auszugeben.

9. System (100) gemäß einem der vorherigen Ansprüche, wobei das System (100) einen Speicher umfasst, der ausgebildet ist, um zumindest den ersten stationären und den ersten mobilen Verdichtungswert zusammen mit der entsprechenden Positionsinformation für die erste Position (48) zu speichern.

10. Baumaschine, insbesondere Verdichtungsmaschine (10), umfassend ein System (100) gemäß einem der Ansprüche 1 bis 9.

11. Verfahren zur Verdichtungsmessung mittels eines Systems (100) gemäß einem der Ansprüche 1 bis 9, mit folgenden Schritten:
Ermitteln zumindest eines ersten stationären Verdichtungswerts zusammen mit einer Positionsinformation für eine erste Position (48) mittels einer ersten Verdichtungsmessvorrichtung (22), die auf einer Verdichtungsmaschine (10) angeordnet ist, unter Verwendung einer gemessenen Beschleunigung einer Messsonde infolge einer Messvibration (15) und/oder einer Beschleunigung eines Verdichtungselements (11, 12) der Verdichtungsmaschine (10), wobei der zumindest erste stationäre Verdichtungswert eine lokale Verdichtung des Untergrunds (51) für die erste Position (48) anzeigt;
Ermitteln zumindest eines ersten mobilen Verdichtungswerts mit zugehöriger Positionsinformation für die erste Position (48) mittels einer zweiten Verdichtungsmessvorrichtung (31), die mobil ist, bei Interaktion oder Kontakt mit dem Untergrund (51); und
Vergleichen des ersten stationären Verdichtungswerts und des ersten mobilen Verdichtungswerts, um einen Korrekturwert zu bestimmen, der von der Abweichung des ersten stationären Verdichtungswerts von dem ersten mobilen Verdichtungswert abhängig ist.

12. Computerprogramm mit einem Programmcode zur Durchführung des Verfahrens nach Anspruch 11, wobei das Computerprogramm auf einem CPU einer Berechnungseinheit eines Verdichtungsmesssystems (100) gemäß einem der Ansprüche 1 bis 9 abläuft.

## Claims

1. System (100) for compaction measurement, comprising:
a first compaction measurement apparatus (22) arrangeable on a compaction machine (10) and configured to determine, on the basis of a measured acceleration of a measurement probe of the first compaction measurement apparatus (22) as a result of a measurement vibration (15) and/or an acceleration of a compaction element (11, 12) of the compaction machine (10), at least one first stationary compaction value that informs about a local compaction of the subsoil (51) or the surfacing for at least a first position (48), and to output the same with associated positional information for the first position (48);
a second compaction measurement apparatus (31) being mobile and configured to identify, upon interaction or contact with the subsoil (51) or the surface, the local compaction of the subsoil (51) or the surfacing for at least the first position (48) and to output, on the basis thereof, at least one first mobile compaction value with associated positional information for the first position (48); wherein the second compaction measurement apparatus (31) is hand-held; wherein the second compaction measurement apparatus (31) has a higher accuracy than the first compaction measurement apparatus (22);
a calculation unit (21) configured to compare the first stationary compaction value and the first mobile compaction value so as to determine a correction value for the first compaction measurement apparatus (22) that depends on the deviation of the first stationary compaction value from the first mobile compaction value;
a position determination apparatus (24, 33) or GNSS apparatus coupled to the first compaction measurement apparatus (22) so as to identify the positional information for the first position (48) if the first compaction measurement apparatus (22) is positioned at the first position (48); and a further position determination apparatus (24, 33) or further GNSS apparatus so as to identify the positional information for the first position (48) if the second compaction measurement apparatus (31) is positioned at the first position (48);
wherein the second compaction measurement apparatus (31) comprises the further position determination device (24, 33) or the further GNSS apparatus as well as a radio interface (31) configured to transmit at least the first mobile compaction value to the calculation unit (21).

2. System (100) according to claim 1, wherein the first compaction measurement apparatus (22) is configured to determine and output a second stationary compaction value for a second position (48); and wherein the second compaction measurement apparatus (31) is configured to determine and output a second mobile compaction value for the second position (48); and/or
wherein the calculation unit (21) is configured to assign, on the basis of the positional information, the first stationary compaction value and the first mobile compaction value to each other and to compare them, and to assign, on the basis of the positional information, the second stationary compaction value and the second mobile compaction value to each other and to compare them.

3. System (100) according to any of the preceding claims, wherein the first compaction measurement apparatus (22) is configured to determine stationary compaction values across a temporal course and to assign the stationary compaction values to a temporal course of a movement trajectory of the compaction machine (10) and/or several positions (48) of the movement trajectory of the compaction machine (10).

4. System (100) according to any of the preceding claims, wherein the second compaction measurement apparatus (31) is configured to identify at least the first mobile compaction value if the second compaction measurement apparatus (31) is placed onto the first position (48); and/or
wherein the second compaction measurement apparatus (31) is configured to identify at least one second mobile compaction value for a second position (48) spaced apart from the first position (48) if the second compaction measurement apparatus (31) is placed onto the second position (48).

5. System (100) according to any of the preceding claims, wherein the second compaction measurement apparatus (31) includes a Troxler probe; and/or wherein the second compaction measurement apparatus (31) includes a gamma radiator and/or a neutron radiator in combination with a counter tube; or
wherein the second compaction measurement apparatus (31) includes a megahertz transmitter, a megahertz receiver, and a processor, wherein the megahertz transmitter is configured to provide an electromagnetic field at 1 MHz or in the range of more than 0.3 MHz, wherein the megahertz receiver is configured to receive a response signal resulting from the electromagnetic field, and wherein the processor is configured to evaluate the response signal so as to identify the first mobile compaction value.

6. System (100) according to any of the preceding claims, wherein the first compaction measurement apparatus (22) includes an interface configured to transmit at least the first stationary compaction value to the calculation unit (21), and/or wherein the calculation unit (21) is coupled to the first compaction measurement apparatus (22).

7. System (100) according to any of the preceding claims, wherein the radio interface (31) is configured to transmit at least the first mobile compaction value also to a further construction machine or to a server.

8. System (100) according to any of the preceding claims, wherein the system (100) includes a temperature sensor (26) configured to identify the temperature of the subsoil (51) or the surfacing at least at the first position (48) and to output the identified temperature value associated with the positional information for the first position (48).

9. System (100) according to any of the preceding claims, wherein the system (100) includes a memory configured to store at least the first stationary and the first mobile compaction value together with the corresponding positional information for the first position (48).

10. Construction machine, in particular a compaction machine (10), including a system (100) according to any of claims 1 to 9.

11. Method for compaction measurement by means of a system (100) according to any of claims 1 to 9, comprising:
identifying at least one first stationary compaction value together with positional information for a first position (48) by means of a first compaction measurement apparatus (22) arranged on a compaction machine (10), using a measured acceleration of a measurement probe as a result of a measurement vibration (15) and/or an acceleration of a compaction element (11, 12) of the compaction machine (10), wherein the at least first stationary compaction value indicates a local compaction of the subsoil (51) for the first position (48);
identifying, upon interaction or contact with the subsoil (51), at least one first mobile compaction value with associated positional information for the first position (48) by means of a second compaction measurement apparatus (31) that is mobile
comparing the first stationary compaction value and the first mobile compaction value so as to determine a correction value that depends on the deviation of the first stationary compaction value from the first mobile compaction value.

12. Computer program with program code for performing the method according to claim 11, wherein the computer program runs on a CPU of a calculation unit of a compaction measurement system (100) according to any of claims 1 to 9.

## Revendications

1. Système (100) de mesure de compactage, aux caractéristiques suivantes:
un premier dispositif de mesure de compactage (22) qui peut être disposé sur une machine de compactage (10) et qui est conçu pour déterminer, partant d'une accélération mesurée d'une sonde de mesure du premier dispositif de mesure de compactage (22) à la suite d'une vibration de mesure (15) et/ou d'une accélération d'un élément de compactage (11, 12) de la machine de compactage (10), au moins une première valeur de compactage stationnaire qui indique un compactage local du sous-sol (51) ou du revêtement pour au moins une première position (48), et pour la sortir avec l'information de position correspondante pour la première position (48);
un deuxième dispositif de mesure de compactage (31) qui est mobile et est conçu pour déterminer, lors de l'interaction ou du contact avec le sous-sol (51) ou avec le revêtement, le compactage local du sous-sol (51) ou du revêtement pour au moins la première position (48) et, partant de cela, pour sortir au moins une première valeur de compactage mobile avec l'information de position correspondante pour la première position (48); où le deuxième dispositif de mesure de compactage (31) est porté à la main; où le deuxième dispositif de compactage (31) présente une précision supérieure à celle du premier dispositif de compactage (22);
une unité de calcul (21) qui est conçue pour comparer la première valeur de compactage stationnaire et la première valeur de compactage mobile pour déterminer une valeur de correction pour le premier dispositif de mesure de compactage (22) qui est fonction de l'écart de la première valeur de compactage stationnaire par rapport à la première valeur de compactage mobile;
un dispositif de détermination de position (24, 33) ou dispositif de GNSS qui couplé au premier dispositif de mesure de compactage (22), destiné à déterminer l'information de position pour la première position (48) lorsque le premier dispositif de mesure de compactage (22) est positionné à la première position (48); et un autre dispositif de détermination de position (24, 33) ou un autre dispositif de GNSS destiné à déterminer l'information de position pour la première position (48) lorsque le deuxième dispositif de mesure de compactage (31) est positionné à la première position (48);
dans lequel le deuxième dispositif de mesure de compactage (31) présente l'autre dispositif de détermination de position (24, 33) ou l'autre dispositif de GNSS ainsi que comporte une interface radio (31) qui est conçue pour transmettre au moins la première valeur de compactage mobile à l'unité de calcul (21).

2. Système (100) selon la revendication 1, dans lequel le premier dispositif de mesure de compactage (22) est conçu pour déterminer et sortir une deuxième valeur de compactage stationnaire pour une deuxième position (48); et dans lequel le deuxième dispositif de mesure de compactage (31) est conçu pour déterminer et sortir une deuxième valeur de compactage mobile pour la deuxième position (48); et/ou
dans lequel l'unité de calcul (21) est conçue pour associer et comparer l'une avec l'autre la première valeur de compression stationnaire et la première valeur de compression mobile à l'aide des informations de position et pour associer et comparer l'une avec l'autre la deuxième valeur de compression stationnaire et la deuxième valeur de compression mobile à l'aide des informations de position.

3. Système (100) selon l'une des revendications précédentes, dans lequel le premier dispositif de mesure de compactage (22) est conçu pour déterminer les valeurs de compactage stationnaires au cours d'une évolution dans le temps et pour associer les valeurs de compactage stationnaires à une évolution dans le temps d'une trajectoire de déplacement de la machine de compactage (10) et/ou plusieurs positions (48) de la trajectoire de déplacement de la machine de compactage (10).

4. Système (100) selon l'une des revendications précédentes, dans lequel le deuxième dispositif de mesure de compactage (31) est conçu pour déterminer au moins la première valeur de compactage mobile lorsque le deuxième dispositif de mesure de compactage (31) est placé sur la première position (48); et/ou
dans lequel le deuxième dispositif de mesure de compactage (31) est conçu pour déterminer au moins une deuxième valeur de compactage mobile pour une deuxième position (48) distante de la première position (48) lorsque le deuxième dispositif de mesure de compactage (31) est placé sur la deuxième position (48).

5. Système (100) selon l'une des revendications précédentes, dans lequel le deuxième dispositif de mesure de compactage (31) comporte une sonde de Troxler; et/ou
dans lequel le deuxième dispositif de mesure de compactage (31) comporte un émetteur gamma et/ou un émetteur de neutrons en combinaison avec un tube compteur; ou
dans lequel le deuxième dispositif de mesure de compactage (31) comporte un émetteur de mégahertz, un récepteur de mégahertz et un processeur, dans lequel l'émetteur de mégahertz est conçu pour fournir un champ électromagnétique à 1 MHz ou dans la plage supérieure à 0,3 MHz, dans lequel le récepteur de mégahertz est conçu pour recevoir un signal de réponse résultant du champ électromagnétique, et dans lequel le processeur est conçu pour évaluer le signal de réponse pour déterminer la première valeur de compactage mobile.

6. Système (100) selon l'une des revendications précédentes, dans lequel le premier dispositif de mesure de compactage (22) comporte une interface qui est conçue pour transmettre au moins la première valeur de compactage stationnaire à l'unité de calcul (21), et/ou dans lequel l'unité de calcul (21) est couplée au premier dispositif de mesure de compactage (22).

7. Système (100) selon l'une des revendications précédentes, dans lequel l'interface radio (31) est conçue pour transmettre au moins la première valeur de compactage mobile également à une autre machine de construction ou à un serveur.

8. Système (100) selon l'une des revendications précédentes, dans lequel le système (100) comporte un capteur de température (26) qui est conçu pour déterminer la température du sous-sol (51) ou du revêtement au moins à la première position (48) et pour sortir une valeur de température déterminée associée à l'information de position pour la première position (48).

9. Système (100) selon l'une des revendications précédentes, dans lequel le système (100) comporte une mémoire qui est conçue pour mémoriser au moins la première valeur de compactage stationnaire et la première valeur de compactage mobile ensemble avec l'information de position correspondante pour la première position (48).

10. Machine de construction, en particulier machine de compactage (10), comportant un système (100) selon l'une des revendications 1 à 9.

11. Procédé de mesure de compactage au moyen d'un système (100) selon l'une des revendications 1 à 9, aux étapes suivantes consistant à:
déterminer au moins une première valeur de compactage stationnaire ensemble avec une information de position pour une première position (48) au moyen d'un premier dispositif de mesure de compactage (22) qui est disposé sur une machine de compactage (10), à l'aide d'une accélération mesurée d'une sonde de mesure à la suite d'une vibration de mesure (15) et/ou d'une accélération d'un élément de compactage (11, 12) de la machine de compactage (10), où l'au moins une première valeur de compactage stationnaire indique un compactage local du sous-sol (51) pour la première position (48);
déterminer au moins une première valeur de compactage mobile avec une information de position associée pour la première position (48) au moyen d'un deuxième dispositif de mesure de compactage (31) qui est mobile, lors de l'interaction ou du contact avec le sous-sol (51); et
comparer la première valeur de compactage stationnaire et la première valeur de compactage mobile pour déterminer une valeur de correction qui est fonction de l'écart de la première valeur de compactage stationnaire par rapport à la première valeur de compactage mobile.

12. Programme d'ordinateur avec un code de programme pour la mise en oeuvre du procédé selon la revendication 11, dans lequel le programme d'ordinateur est exécuté sur une CPU d'une unité de calcul d'un système de mesure de compactage (100) selon l'une des revendications 1 à 9.
